# FASCICULE DE BREVET EUROPEEN

(11) **EP 0 854 688 B1**
(45) Date de publication et mention de la délivrance du brevet: **25.06.2003**
(21) Numéro de dépôt: 96930206.6
(22) Date de dépôt: 04.09.1996
(51) Int. Cl.: A61B 5/04, A61B 5/0428

(54) **DISPOSITIF D'ELECTRO-PHYSIOLOGIE**
ELEKTROPHYSIOLOGISCHE VORRICHTUNG
ELECTROPHYSIOLOGICAL DEVICE

(30) Priorité: 08.09.1995 FR 9510565
(43) Date de publication de la demande: 29.07.1998
(73) Titulaire: CENTRE NATIONAL DE LA RECHERCHE SCIENTIFIQUE, 75016 Paris Cédex (FR)
(72) Inventeur: RIPOCHE, André, F-94400 Vitry-sur-Seine (FR); BAUDONNIERE, Pierre-Marie, F-75015 Paris (FR)
(74) Mandataire: Plaçais, Jean-Yves
(86) Numéro de dépôt international: FR9601351
(87) Numéro de publication internationale: WO97008988

(56) Documents cités:
- EP-A- 0 165 141
- US-A- 3 880 146
- US-A- 4 817 627
- MEDICAL AND BIOLOGICAL ENGINEERING AND COMPUTING, vol. 32, no. 4, Juillet 1994, STEVENAGE GB, pages 459-461, XP000444898 H. IGUCHI ET AL.: "Wearable electroencephalograph system with preamplified electrodes"
- FUNKSCHAU, vol. 57, no. 21, Octobre 1985, MUNCHEN DE, pages 76-78, XP002004729 M. HANDWERKER: "Bio-Verstärker: Signale vom Herzen"

## Description

L'invention concerne le domaine de l'électro-physiologie, et notamment la détection par des électrodes, en milieu difficile, et en vue de leur enregistrement, de signaux électro-physiologiques, de type électromagnétique, émis par un sujet.

Elle concerne plus particulièrement les dispositifs du type comprenant un équipage capteur adaptable à un sujet, et muni d'un jeu d'électrodes aptes à détecter des signaux électromagnétiques, pour les délivrer sous forme de signaux de sortie à des entrées de moyens de traitement aptes, par ailleurs, à les mettre en forme avant mémorisation.

Un tel jeu connu par example du document US-A-3 880 146, comprend généralement des électrodes de travail comportant au moins une électrode d'acquisition installée en position choisie dans ou sur l'équipage capteur, ainsi qu'une électrode supplémentaire à relier aux moyens de traitement en tant que masse flottante.

On entend par équipage capteur, par exemple un casque muni d'électrodes destinées à détecter l'activité cérébrale, du type électro-encéphalogramme (EEG), ou bien un appareil destiné à détecter l'activité musculaire, du type électromyogramme (EMG), ou bien encore un appareil destiné à détecter l'activité cardiaque, du type électrocardiogramme (ECG), ou plus généralement tout appareil d'acquisition de signaux électro-physiologiques.

Par ailleurs, on entend par sujet, tout être vivant (humain ou animal), qu'il soit cliniquement malade, et dans ce cas il s'agit d'un patient, ou bien qu'il soit cliniquement sain.

Le Demandeur s'est aperçu que dans certaines conditions d'utilisation, les signaux détectés par les électrodes d'un tel équipage capteur présentaient un rapport signal sur bruit (S/B) qui était de l'ordre de l'unité, et par conséquent n'étaient pas réellement représentatifs de l'activité recherchée.

C'est particulièrement le cas des examens destinés à détecter des signaux électriques, d'une part, de très faible amplitude, typiquement de l'ordre de quelques microvolts, sur des sujets soumis à des déplacements imposés, comme par exemple lors d'une stimulation de leur système vestibulaire (afin de recueillir des potentiels évoqués vestibulaires), et/ou d'autre part, sur des sujets qui ne sont pas facile à maintenir immobile, comme par exemple les bébés, les hyperkinétiques ou bien les malades atteints de la maladie de Parkinson.

C'est aussi le cas, lors d'enregistrements dans des environnements bruités d'un point de vue électro-magnétique, même lorsque le sujet est immobile.

Le Demandeur s'est aperçu que la cause principale de cette effet de bruitage des signaux pouvait être attribuée à l'effet couplé de l'impédance des électrodes (typiquement quelques kiloohms) et des boucles électriques que forment les câbles de liaison entre ces électrodes et les moyens de traitement. Ces boucles, lorsqu'elles sont soumises à des déplacements dans un champ électromagnétique continu présentent en leurs bornes une tension parasite induite assimilable à du bruit électrique.

Une telle tension est d'autant plus gênante qu'elle est, dans le cas des déplacements imposés, en phase avec les signaux représentatifs de l'activité recherchée, et son amplitude est d'autant plus grande que la dimension de la boucle est grande et que l'impédance de cette boucle, et par conséquent des électrodes, est grande.

La présente invention a pour but d'améliorer la situation, en apportant une solution à ce problème de bruitage des mesures qui interdit leur analyse.

L'invention propose à cet effet un dispositif du type défini précédemment, et dans lequel, d'une part, la sortie de chaque électrode de travail est équipée d'un premier moyen adaptateur d'impédance, et d'autre part, on prévoit une alimentation électrique commune pour ces premiers moyens adaptateurs d'impédance, ainsi qu'un circuit propre à maintenir un potentiel électrique intermédiaire, lié à cette alimentation, au potentiel électrique de l'électrode supplémentaire, lequel dépend bien entendu de l'endroit où se trouve placée ladite électrode supplémentaire. Ce potentiel intermédiaire est donc compris entre les valeurs respectives des potentiels des deux bornes extrémales de l'alimentation.

Ainsi, en équipant la sortie de chaque électrode de travail d'un moyen adaptateur d'impédance, on réduit très notablement leur impédance de sortie, ce qui tend à supprimer l'effet induit par les déplacements du sujet. Par ailleurs, le circuit étant connecté à un point intermédiaire de l'alimentation, cela permet de définir une masse flottante qui servira de masse de référence au dispositif et aux moyens de traitement.

Selon une autre caractéristique de l'invention, la sortie de l'électrode supplémentaire est également équipée d'un second moyen adaptateur d'impédance.

De façon particulièrement avantageuse, les premiers et second moyens adaptateurs d'impédance sont réalisés à l'aide d'un amplificateur opérationnel, lequel, de par sa très petite taille, peut être facilement installé à la sortie de chaque électrode, ou bien intégré directement dans l'électrode, sans que cela n'entraîne une augmentation sensible du poids de l'équipage capteur.

Selon encore une autre caractéristique de l'invention, la sortie de chaque amplificateur opérationnel est couplée à l'entrée inverseuse de celui-ci.

Ainsi, en établissant une contre-réaction, le gain en tension de chaque amplificateur opérationnel devient sensiblement égal à l'unité pour les signaux appliqués à son entrée non inverseuse. Chaque amplificateur opérationnel est ainsi transformé en suiveur de tension d'impédance de sortie quasi nulle.

De préférence, les gains respectifs des premiers moyens adaptateurs d'impédance sont sensiblement égaux, ce qui permet d'éviter une renormalisation des signaux.

Dans un premier mode de réalisation du dispositif, la sortie de chaque électrode est connectée à l'entrée non inverseuse de l'amplificateur opérationnel correspondant, lequel amplificateur opérationnel présente une sortie connectée à l'entrée correspondante des moyens de traitement. Le potentiel de la sortie de l'électrode supplémentaire est le potentiel de l'électrode supplémentaire, et par conséquent, selon l'invention, il est égal au potentiel intermédiaire.

Dans un second mode de réalisation du dispositif, la sortie de chaque électrode de travail est connectée à l'entrée non inverseuse de l'amplificateur opérationnel correspondant, lequel présente une sortie connectée à l'entrée correspondante des moyens de traitement, tandis que la sortie de l'électrode supplémentaire est connectée à la sortie de l'amplificateur opérationnel correspondant, lequel amplificateur opérationnel présente une entrée non inverseuse connectée à l'entrée correspondante des moyens de traitement et maintenue au potentiel électrique intermédiaire, lié à l'alimentation, par l'intermédiaire du circuit de l'électrode supplémentaire.

Le circuit de l'électrode supplémentaire comprend, préférentiellement, de première et seconde résistances, de caractéristiques identiques, respectivement entre les première et seconde bornes d'extrémité de l'alimentation et l'entrée non inverseuse de l'amplificateur opérationnel correspondant à l'électrode supplémentaire.

Mais, le circuit de l'électrode supplémentaire peut être réalisé différemment, l'entrée non inverseuse de l'amplificateur opérationnel étant directement connectée au point milieu de l'alimentation.

Dans chacun de ces deux modes, il est possible de maintenir l'électrode supplémentaire à un potentiel électrique intermédiaire lié à un point intermédiaire de l'alimentation, lequel est en fait le point milieu, de potentiel sensiblement égal à zéro volt. Par conséquent, la masse flottante qui fixe le potentiel de référence des moyens de traitement est également sensiblement au potentiel zéro volt.

L'amplificateur opérationnel qui équipe l'électrode supplémentaire est de préférence du type bipolaire ou du type à effet de champ. Il en va de même pour chaque amplificateur opérationnel équipant une électrode de travail.

Bien entendu, on peut envisager que l'amplificateur opérationnel de l'électrode supplémentaire soit d'un type donné, tandis que les amplificateurs opérationnels des électrodes de travail sont tous de l'autre type.

Lorsque les amplificateurs opérationnels des électrodes de travail sont du type à effet de champ, il est préférable que le dispositif selon l'invention comprenne, entre chaque borne d'extrémité de l'alimentation et l'entrée non inverseuse de chaque amplificateur opérationnel correspondant à chaque électrode de travail, respectivement une première et une seconde diodes polarisées en inverse et de caractéristiques identiques.

Cela limite ainsi très notablement les possibilités de claquage électrique d'un amplificateur opérationnel, ce qui le rendrait inopérant et par conséquent interdirait l'analyse des signaux détectés par l'électrode correspondante.

Préférentiellement, les électrodes de travail comportent également au moins une électrode de référence destinée(s) à fournir un ou plusieurs potentiels de référence qui serviront lors du traitement des signaux électriques issus des électrodes d'acquisition.

Bien entendu, ces électrodes de référence étant des électrodes de travail, elles sont équipées du même type d'adaptateur d'impédance que les électrodes d'acquisition.

Mais il est clair que de telles électrodes de référence ne sont pas indispensables, si, d'une part, le nombre d'électrodes est suffisant, et si, d'autre part, les moyens d'acquisition sont aptes à calculer un potentiel fictif de référence à partir de l'ensemble des potentiels délivrés par les électrodes d'acquisition. Ce potentiel fictif remplacerait alors le ou les potentiels de référence délivrés normalement par la ou les électrodes de référence.

En conséquence, en variante, on peut prévoir un dispositif sans électrodes de référence mais équipé d'au moins trois électrodes d'acquisition, et dont les moyens de traitement sont aptes à calculer un potentiel fictif de référence à partir des signaux délivrés par chaque électrode d'acquisition.

Ce potentiel fictif de référence est représentatif de la moyenne des potentiels délivrés par chaque électrode d'acquisition.

Selon encore une autre caractéristique de l'invention, l'alimentation est constituée d'une pile, ou de plusieurs piles mises en série, ou bien encore d'accumulateurs rechargeables, dont la différence de potentiel entre les deux bornes d'extrémité est sensiblement égale à 9 Volts.

Ce mode d'alimentation permet d'une part, de sécuriser le montage sur le sujet, et d'autre part d'éviter les parasites électriques qui sont habituellement générés par un convertisseur continu-continu bien que son utilisation soit tout à fait envisageable.

Dans une application à l'EEG, l'équipage capteur du dispositif proposé est un casque d'électro-physiologie adaptable sur la tête du sujet. Les signaux détectés par les électrodes d'acquisition sont alors des ondes d'activité cérébrale provenant de régions identifiables du cerveau.

Il est ainsi possible, par exemple, de détecter les potentiels évoqués de sujets soumis à des stimulations vestibulaires, ou bien d'étudier l'activité cérébrale de sujets qu'il est difficile d'immobiliser.

D'autres caractéristiques et avantages de l'invention apparaîtront à l'examen de la description détaillée ci-après, et des dessins annexés, sur lesquels :
- la figure 1 est un schéma illustrant les principaux éléments du dispositif selon l'invention dans un mode de réalisation destiné à l'EEG, et
- la figure 2 est un schéma illustrant les moyens adaptateurs d'impédance en sortie des électrodes de l'équipage capteur selon l'invention, dans un mode de réalisation préféré.,

Les dessins annexés sont, pour l'essentiel, de caractère certain. En conséquence, ils font partie intégrante de la description et pourront non seulement servir à compléter celle-ci, mais aussi contribuer à la définition de l'invention le cas échéant.

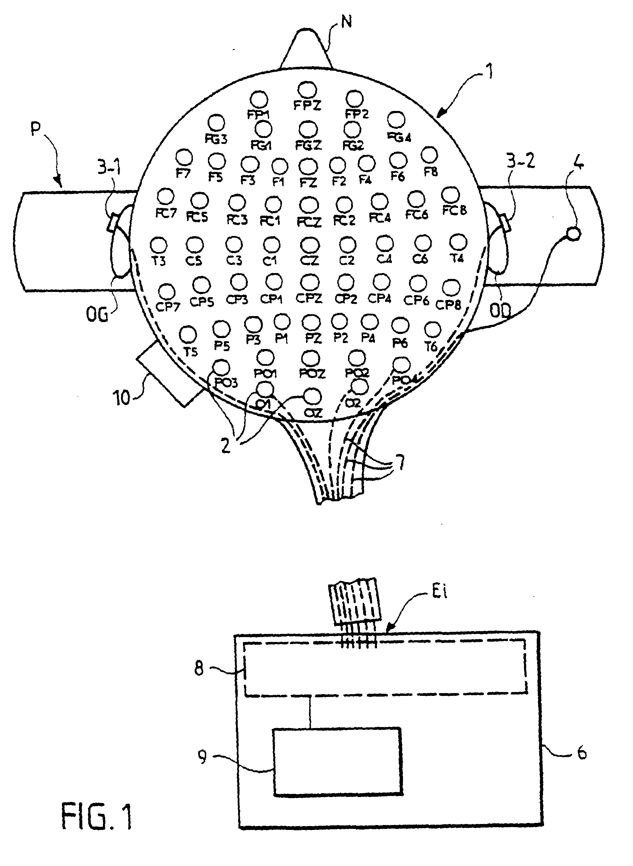

On se réfère tout d'abord à la figure 1 pour décrire un dispositif d'électro-physiologie selon l'invention.

Dans toute la description qui suit, on considérera que l'équipage capteur est un casque d'électro-physiologie 1 destiné à détecter des signaux électriques représentatifs de potentiels évoqués d'un sujet P, notamment d'un homme, soumis à des stimulations vestibulaires. Mais il est clair que l'invention proposée s'applique à de nombreux autres domaines.

Le casque d'électro-physiologie 1 est réalisé dans un matériau souple, et sa forme est sensiblement sphérique, ce qui permet de l'adapter sur la tête d'un sujet P de type humain, lequel est installé, pour ce type de stimulations, dans un fauteuil (non représenté).

Néanmoins, tout autre casque ou montage équipé d'électrodes aptes à détecter des ondes d'activité cérébrale sur le scalp d'un sujet pourra être utilisé, et particulièrement des électrodes collées.

Ce casque 1 comprend un jeu d'électrodes comportant des électrodes de travail 2 et 3, ainsi qu'une électrode supplémentaire 4.

Les électrodes de travail comprennent, d'une part, des électrodes d'acquisition 2-j (j = 1 à n) destinées à détecter les signaux électriques représentatifs des potentiels évoqués émis par certaines régions du cerveau en réponse aux stimulations, et d'autre part, une ou deux, ou même trois, électrodes de référence 3-k (k = 1 à 3).

On décrit ici une acquisition comprenant une électrode de référence installée sur le nez N du patient, ou deux électrodes de référence placées respectivement sur ses oreilles droites OD et gauche OG pour calculer la moyenne de leurs potentiels respectifs, ou pour utiliser ceux-ci séparément. Mais il est également possible d'utiliser toute autre partie du corps du patient comme potentiel de référence.

Ces électrodes 3-k sont destinées à fournir un potentiel de référence représentatif à chaque instant du potentiel de surface à proximité des électrodes, de sorte que l'on puisse déduire les vrais signaux d'origine vestibulaire, par soustraction entre les signaux électriques détectés par les électrodes d'acquisition 2-j et les signaux électriques détectés par les électrodes de référence 3-k.

Chaque électrode d'acquisition 2 est installée dans la structure du casque 1 en un endroit choisi, où elle définit une zone cérébrale sur le scalp du sujet, chaque zone cérébrale étant associée à au moins une région identifiable du cerveau, supposée être une projection vestibulaire.

Selon la précision de l'examen, ce casque peut comporter de une à n électrodes, le nombre n n'étant pas limitatif.

Sur la figure 1 sont représentées les positions respectives de 61 électrodes en référence aux oreilles droite OD et gauche OG, ainsi qu'au nez N d'un sujet de type humain.

L'électrode supplémentaire 4 est généralement installée sur la joue ou l'épaule du sujet P. Elle permet de fixer la valeur du potentiel de la masse flottante du dispositif. On reviendra plus loin sur celle-ci.

La sortie 5 de chaque électrode 2 à 4 est connectée à une entrée Ei (i = 2 à 4) des moyens de traitement 6 par l'intermédiaire d'un câble électrique 7 d'une longueur d'environ 1 mètre à 1,5 mètres.

Ces moyens de traitement 6 comprennent des moyens de mise en forme 8 destinés au moins à amplifier chaque signal reçu de façon indépendante, ainsi que des moyens de mémorisation 9 destinés à stocker les signaux ainsi amplifiés en vue de leur analyse ultérieure.

Bien entendu, la mise en forme peut également comprendre un ou plusieurs filtrages, ainsi qu'une numérisation avant mémorisation.

Par ailleurs, les moyens de traitement peuvent être agencés de sorte qu'ils puissent afficher en temps réel certains au moins des signaux représentatifs des potentiels évoqués émis en réponse à une stimulation, après soustraction des potentiels de référence détectés par les électrodes 3-k.

Le traitement peut également comprendre une étape, avant affichage, dans laquelle on recherche les potentiels évoqués en relation avec la loi de mouvement choisie pour stimuler le système vestibulaire du sujet.

De même, afin de déterminer avec un maximum de précision les régions du cerveau impliquées dans la réponse vestibulaire à la stimulation, on pourra effectuer une analyse des potentiels évoqués par combinaison linéaire de certaines électrodes d'acquisition 2-j du casque 1.

Enfin, le casque, comme tout autre dispositif d'enregistrement, peut également comprendre des électrodes (non représentées sur les figures) destinées à être fixées au voisinage des paupières du sujet afin de recueillir les mouvements verticaux et horizontaux de ses yeux. Bien entendu, de telles électrodes sont connectées aux moyens de traitement 6.

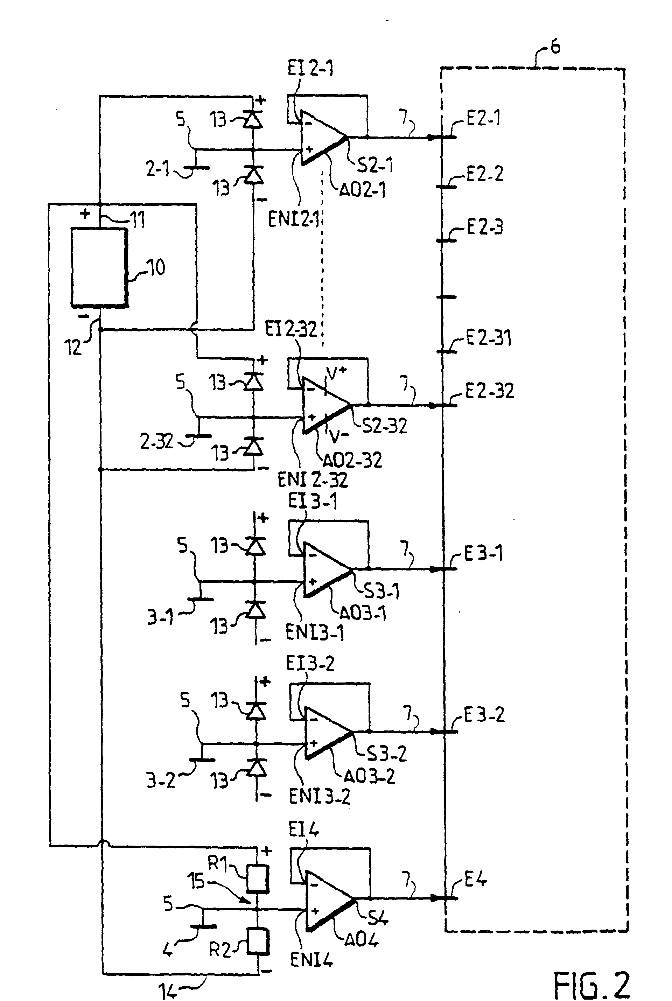

On se réfère maintenant à la figure 2, pour décrire plus en détail la sortie 5 des électrodes 2 à 4 du casque 1.

Les signaux détectés par les électrodes d'acquisition 2-j (j = 1 à 32) sont délivrés aux moyens de traitement sous forme de différence de potentiel dont l'amplitude dépend, d'une part, du nombre de neurones impliqués dans la réponse, et d'autre part, de la profondeur à laquelle se trouve la partie du cerveau qui émet. Ces différences de potentiel sont donc, généralement, de très faible intensité, typiquement quelques microvolts.

Les électrodes 2 à 4 utilisées par l'homme de l'art présentent une impédance de sortie élevée, typiquement quelques kiloohms. En fait, leur impédance est due au contact avec le scalp du sujet, lequel est réalisé au moyen d'une pâte conductrice ou d'un gel conducteur.

Comme il a été expliqué dans l'introduction, le déplacement des câbles de liaison 7 dans un champ électromagnétique continu induit une différence de potentiel parasite dans ces câbles, laquelle est d'autant plus grande que l'impédance de sortie de l'électrode est grande.

Pour supprimer ces tensions parasites en phase avec les stimulations, il existe deux solutions. La première solution consiste à raccourcir au maximum la longueur des câbles de liaison 7. La seconde solution consiste à réduire au maximum l'impédance de sortie des électrodes.

Il est clair que la première solution n'est pas envisageable, puisqu'elle imposerait d'installer une partie des moyens de traitement, et notamment la partie amplification 8, sur le casque 1, ce qui augmenterait très notablement le poids dudit casque et pourrait même éventuellement perturber les mesures.

En conséquence, le Demandeur a eu l'idée particulièrement avantageuse d'installer en sortie de chaque électrode 2 à 4 un moyen adaptateur d'impédance Aoi (i = 2 à 4) destiné à réduire leur impédance de sortie à une valeur de l'ordre de l'ohm. Ces moyens adaptateurs d'impédance n'ont pas été représentés sur la figure 1 pour des raisons d'encombrement.

Chaque moyen adaptateur d'impédance AOi est constitué d'un amplificateur opérationnel de type à effet de champ, comme celui proposé par la Société Analog Device sous la référence AD795, ou bien de type bipolaire, comme celui proposé par la Société Linear Technology sous la référence LT1012.

Chaque amplificateur opérationnel est monté en suiveur de tension (ou en boucle fermée), de sorte que son gain soit sensiblement égal à 1 et par conséquent son impédance de sortie de l'ordre de l'ohm. Pour ce faire, sa sortie Si (i = 2 à 4) est directement connectée à son entrée dite inverseuse EIi.

Un mode de réalisation est préféré, mode dans lequel la sortie 5 de chacune des électrodes 2 à 4 est connectée à l'entrée non inverseuse ENIi (i = 2 à 4) de l'amplificateur opérationnel correspondant AOi, lequel présente une sortie Si connectée à l'entrée correspondante Ei des moyens de traitement 6.

Dans ce mode, le gain de chaque amplificateur opérationnel AOi est égal à +1.

Pour éviter tout nouveau parasitage qui pourrait survenir en raison de la liaison entre l'électrode et l'amplificateur opérationnel il est préférable que ledit amplificateur opérationnel soit directement connecté à l'électrode, ou mieux encore, qu'il soit directement intégré à l'électrode lors de sa fabrication, ou bien encore à la structure du casque.

Chacun de ces amplificateurs opérationnels AOi (i = 2 à 4) nécessite une alimentation continue pour fonctionner. En conséquence, on prévoit une alimentation 10 que l'on installe, par exemple, sur le casque 1. Bien entendu, cette alimentation peut être installée en tout autre endroit pas trop éloigné de la tête du patient. Les entrées V+ et V-d'alimentation d'un amplificateur opérationnel ne sont représentées que sur A02-32 pour des raisons d'encombrement, mais il est clair que chaque amplificateur opérationnel est alimenté de la même façon. De préférence, l'alimentation est réalisée à partir d'une ou plusieurs piles mises en série, ou bien à l'aide d'accumulateurs rechargeables. La différence de potentiel aux bornes de l'alimentation est de préférence égale à 9 volts, le premier point d'extrémité 11 étant à un potentiel supérieur de +4,5 volts, tandis que le second point d'extrémité 12 est à un potentiel inférieur de -4,5 volts.

Cette solution est préférée à celle consistant à utiliser un convertisseur de tension continu-continu, qui génère parfois des parasites. Par ailleurs, une alimentation par pile assure une meilleure isolation par rapport aux circuits environnants et est plus sûre pour le patient qui est connecté à cette alimentation par l'intermédiaire des électrodes, comme on le verra plus loin.

Lorsque les amplificateurs opérationnels AO2-j et AO3-k sont du type à effet de champ, il est préférable, pour éviter les claquages électriques, que leur entrée non inverseuse ENIi (i = 2 ou 3) soit protégée. Pour ce faire, d'une part, on relie, par l'intermédiaire d'une première diode 13 polarisée en inverse, le premier point d'extrémité 11 de l'alimentation 10 à l'entrée non inverseuse ENI2-j et ENI3-k de chaque amplificateur opérationnel correspondant à chaque électrode de travail 2-j et 3-k, et d'autre part, on relie, par l'intermédiaire d'une seconde diode 13 polarisée en inverse, le second point d'extrémité 12 de ladite alimentation 10 à l'entrée non inverseuse ENI2-j et ENI3-k de chaque amplificateur opérationnel correspondant à chaque électrode de travail 2-j et 3-k. Ces premières et secondes diodes doivent être de caractéristiques sensiblement identiques.

De préférence, ce sont des diodes à très faible courant inverse, typiquement de quelques picoampères, comme par exemple les diodes du type PAD5 (pour Pico Ampère Diode à courant inverse maximum de 5 pA).

Ce montage particulièrement avantageux permet de bloquer les charges électriques afin que l'entrée non inverseuse de chaque amplificateur opérationnel ainsi équipé ne soit pas soumise à des tensions supérieures aux tensions respectives des points d'extrémité 11 et 12 de l'alimentation (+/- 4,5 V).

Par contre, quel que soit le type de l'amplificateur opérationnelle AO4 connecté à l'électrode supplémentaire 4, il est indispensable de prévoir un circuit 14 permettant de connecter son entrée non inverseuse ENI4 à l'alimentation 10. En effet, cette électrode 4 est connectée aux moyens de traitement 6 et au patient, afin de définir une masse flottante pour l'ensemble du dispositif. De préférence, cette masse flottante est un potentiel électrique d'environ zéro volt.

Une première solution consiste à connecter directement l'entrée non inverseuse ENI4 de AO4 à un point intermédiaire ou point milieu de l'alimentation 10. De la sorte, si ce point milieu correspond effectivement au potentiel zéro volt alors l'entrée non inverseuse ENI4 se trouve au potentiel zéro volt, tout comme la masse des moyens de traitement 6 puisque celle-ci est connectée à la sortie de AO4 qui fonctionne en tant que suiveur de tension de gain +1.

Une seconde solution consiste, d'une part, à relier, par l'intermédiaire d'une première résistance R1, le premier point d'extrémité 11 de l'alimentation 10 à l'entrée non inverseuse ENI4 de l'amplificateur opérationnel AO4, et d'autre part, à relier, par l'intermédiaire d'une seconde résistance R2, le second point d'extrémité 12 de ladite alimentation 10 à l'entrée non inverseuse ENI4 de l'amplificateur opérationnel AO4. Ces premières et secondes résistances doivent être de caractéristiques sensiblement identiques afin d'éviter un déséquilibre électrique.

Ainsi, on crée un point intermédiaire 15 de potentiel sensiblement égal à zéro (aux fluctuations des caractéristiques des résistances près), lequel va définir grâce à AO4 la masse flottante de référence du dispositif, comme indiqué ci-avant.

Cette seconde solution est préférée car elle permet d'éviter d'utiliser un câble de liaison additionnel non protégé entre l'électrode supplémentaire et le point milieu de l'alimentation, lequel nécessiterait par ailleurs, pour être créé, une adaptation de l'alimentation.

Un autre mode de réalisation peut être envisagé pour l'adaptation d'impédance de l'électrode supplémentaire AO4, mode dans lequel la sortie 5 de l'électrode supplémentaire 4 est connectée à la sortie S4 de AO4, lequel présente une entrée non inverseuse ENI4 connectée à l'entrée correspondante E4 des moyens de traitement 6, ainsi qu'au point milieu de l'alimentation ou à un potentiel intermédiaire défini comme précédemment par deux résistances de caractéristiques identiques installées respectivement entre les extrémités 11 et 12 de l'alimentation.

Dans la description ci-dessus, on a décrit un dispositif adapté à la tête d'un sujet de type humain, mais il est clair qu'un tel dispositif de test peut être adapté à l'examen de tout être vivant. Cela implique que d'autres types de casque d'électro-physiologie peuvent être utilisés sans pour autant sortir du cadre de l'invention.

De même, l'invention ne se limite pas aux casques d'électrophysiologie, mais elle concerne tout type d'équipage capteur muni d'électrodes, comme par exemple les appareils servant pour les analyses musculaires (EMG) ou cardiaques (ECG).

## Revendications

1. Dispositif d'électro-physiologie, du type comprenant un équipage capteur (1) adaptable à un sujet (P), et muni d'un jeu d'électrodes (2-4) aptes à détecter des signaux électromagnétiques, pour les délivrer sous forme de signaux de sortie à des entrées (E) de moyens de traitement (6,8,9) aptes, par ailleurs, à les mettre en forme avant mémorisation, ledit jeu comprenant des électrodes de travail (2,3) comportant au moins une électrode d'acquisition (2) en position choisie, ainsi qu'une électrode supplémentaire (4) à relier aux moyens de traitement en tant que masse flottante,
**caractérisé en ce que** la sortie (S2-j,S3-k; j=1 à n, k=1 à 3) de chaque électrode de travail (2-j,3-k) est équipée d'un premier moyen adaptateur d'impédance (AO2-j,AO3-k), et
**en ce qu'**il comprend une alimentation électrique (10) commune pour ces premiers moyens adaptateurs d'impédance (AO2,AO3), ainsi qu'un circuit (14) propre à maintenir un potentiel électrique intermédiaire, lié à cette alimentation (10), au potentiel électrique de l'électrode supplémentaire (E4).

2. Dispositif selon la revendication 1, **caractérisé en ce que** la sortie (S4) de l'électrode supplémentaire (4) est équipée d'un second moyen adaptateur d'impédance (AO4) alimenté également par l'alimentation (10).

3. Dispositif selon la revendication 2, **caractérisé en ce que** chacun des premiers (AO2,AO3) et second (AO4) moyens adaptateurs d'impédance est un amplificateur opérationnel comprenant des entrées inverseuse (EIi; i=2 à 4) et non inverseuse (ENIi), ainsi qu'une sortie (Si).

4. Dispositif selon la revendication 3, **caractérisé en ce que** la sortie (Si) de chaque amplificateur opérationnelle (AOi) est couplée à l'entrée inverseuse (EIi) de celui-ci.

5. Dispositif selon l'une des revendications 1 à 4, **caractérisé en ce que** les gains respectifs desdits premiers moyens adaptateurs d'impédance sont sensiblement égaux.

6. Dispositif selon l'une des revendications 3 à 5, **caractérisé en ce que** chaque électrode de travail (2,3) présente une sortie (5) connectée à l'entrée non inverseuse (ENI2-j, ENI3-k) de l'amplificateur opérationnel correspondant (AO2-j,AO3-k), lequel présente une sortie (S2-j,S3-k) connectée à l'entrée (E2-j,E3-k) correspondante des moyens de traitement (6,8,9).

7. Dispositif selon l'une des revendications 3 à 6, **caractérisé en ce que** l'électrode supplémentaire (4) présente une sortie (5) connectée à l'entrée non inverseuse (ENI4) de l'amplificateur opérationnel correspondant (AO4), lequel présente une sortie (S4) connectée à l'entrée correspondante (E4) des moyens de traitement (6,8,9).

8. Dispositif selon l'une des revendications 3 à 6, **caractérisé en ce que** l'électrode supplémentaire (4) présente une sortie (5) connectée à la sortie (S4) de l'amplificateur opérationnel correspondant (AO4), lequel présente une entrée non inverseuse (ENI4) connectée à l'entrée correspondante (E4) des moyens de traitement (6,8,9) et maintenue au potentiel électrique intermédiaire lié à l'alimentation par l'intermédiaire du circuit de l'électrode supplémentaire (14).

9. Dispositif selon l'une des revendications 3 à 8, **caractérisé en ce que** le circuit de l'électrode supplémentaire (14) comprend une première résistance (R1) entre une première borne d'extrémité (11) de l'alimentation (10) et l'entrée non inverseuse (ENI4) de l'amplificateur opérationnel (AO4) correspondant à l'électrode supplémentaire (4), ainsi qu'une seconde résistance (R2), de valeur égale à la première résistance, entre une seconde borne d'extrémité (12) de ladite alimentation (10) et l'entrée non inverseuse (ENI4) dudit amplificateur opérationnel (AO4) correspondant à l'électrode supplémentaire (4).

10. Dispositif selon l'une des revendications 3 à 8, **caractérisé en ce que** l'entrée non inverseuse de l'amplificateur opérationnel est directement connectée au point milieu de l'alimentation.

11. Dispositif selon l'une des revendications 3 à 10, **caractérisé en ce que** l'amplificateur opérationnel (AO4) équipant l'électrode supplémentaire (4) est du type bipolaire.

12. Dispositif selon l'une des revendications 3 à 10, **caractérisé en ce que** l'amplificateur opérationnel (AO4) équipant l'électrode supplémentaire (4) est du type à effet de champ.

13. Dispositif selon l'une des revendications 3 à 12, **caractérisé en ce que** chaque amplificateur opérationnel (AO2-j,AO3-k) équipant une électrode de travail (2-j,3-k) est du type bipolaire.

14. Dispositif selon l'une des revendications 3 à 12, **caractérisé en ce que** chaque amplificateur opérationnel (AO2-j,AO3-k) équipant une électrode de travail (2-j,3-k) est du type à effet de champ.

15. Dispositif selon la revendication 14, **caractérisé en ce qu'**il comprend, d'une part, entre la première borne d'extrémité (11) de l'alimentation (10) et l'entrée non inverseuse (ENI2-j,ENI3-k) de chaque amplificateur opérationnel (AO2-j,AO3-k) correspondant à chaque électrode de travail (2-j,3-k), une première diode (13) polarisée en inverse, et d'autre part, entre la seconde borne d'extrémité (12) de ladite alimentation (10) et l'entrée non inverseuse (ENI2-j,ENI3-k) de chaque amplificateur opérationnel (AO2-j,AO3-k) correspondant à chaque électrode de travail (2-j,3-k), une seconde diode (13) polarisée en inverse et de caractéristiques identiques à celles des premières diodes.

16. Dispositif selon l'une des revendications précédentes, **caractérisé en ce que** les électrodes de travail (2,3) comprennent au moins une électrode de référence (3).

17. Dispositif selon l'une des revendications 1 à 15, **caractérisé en ce que** les électrodes d'acquisition sont au moins trois, et **en ce que** les moyens de traitement sont aptes à calculer un potentiel fictif de référence à partir des signaux délivrés par chaque électrode d'acquisition.

18. Dispositif selon l'une des revendications précédentes, **caractérisé en ce que** l'alimentation (10) est constituée d'une pile, ou de plusieurs piles mises en série, ou bien encore d'accumulateurs rechargeables, dont la différence de potentiel à ses bornes (11,12) est sensiblement égale à 9 Volts.

19. Dispositif selon l'une des revendications 1 à 17, **caractérisé en ce que** l'alimentation (10) est constituée d'un convertisseur continu-continu.

20. Dispositif selon l'une des revendications 1 à 19, **caractérisé en ce que** l'équipage capteur (1) est un casque d'électro-physiologie adaptable sur le crâne du sujet (P), et **en ce que** les signaux détectés par les électrodes d'acquisition sont des ondes d'activité cérébrale provenant de régions identifiables du cerveau.

## Patentansprüche

1. Elektrophysiologische Vorrichtung, enthaltend eine Aufnehmeranordnung (1), die an ein Subjekt (P) anpassbar und mit einem Satz Elektroden (2-4) versehen ist, die dazu eingerichtet sind, elektromagnetische Signale aufzunehmen, um diese in Form von Ausgangssignalen an die Eingänge (E) von Verarbeitungseinrichtungen (6,8,9) abzugeben, die unter anderem dazu eingerichtet sind, die Signale vor Speicherung aufzubereiten, wobei der genannte Elektrodensatz Arbeitselektroden (2,3) enthält, die wenigstens eine Erfassungselektrode (2) an vorgegebener Position sowie eine Zusatzelektrode (4) zur Verbindung mit den Verarbeitungseinrichtungen als schwimmende Masse aufweist,
**dadurch gekennzeichnet, dass** der Ausgang (S2-j,S3-k; j=1 bis n, k=1 bis 3) einer jeden Arbeitselektrode (2-j,3-k) mit einer ersten Impedanzanpasseinrichtung (AO2-j, AO3-k) versehen ist,
dass sie eine elektrische Versorgungseinrichtung (10), die den ersten Impedanzanpasseinrichtungen (AO2,AO3) gemeinsam ist, sowie eine Schaltung (14) enthält, die ein elektrisches Zwischenpotentials, das mit dieser Versorgungsquelle (10) verbunden ist, auf dem elektrischen Potential der Zusatzelektrode (4) zu halten.

2. Vorrichtung nach Anspruch 1, **dadurch gekennzeichnet, dass** der Ausgang (S4) der Zusatzelektrode (4) mit einer zweiten Impedanzanpasseinrichtung (AO4) versehen ist, die ebenfalls von der Versorgungseinrichtung (10) versorgt ist.

3. Vorrichtung nach Anspruch 2, **dadurch gekennzeichnet, dass** jede der ersten (AO2,AO3) und zweiten (AO4) Impedanzanpasseinrichtungen ein Operationsverstärker ist, der invertierende Eingänge (Eli; i=2 bis 4) und nicht-invertierende Eingänge (ENIi) sowie einen Ausgang (Si) aufweist.

4. Vorrichtung nach Anspruch 3, **dadurch gekennzeichnet, dass** der Ausgang (Si) eines jeden Operationsverstärkers (AOi) mit dem invertierenden Eingang (Eli) desselben verbunden ist.

5. Vorrichtung nach einem der Ansprüche 1 bis 4, **dadurch gekennzeichnet, dass** die jeweiligen Verstärkungen der ersten Impedanzanpasseinrichtungen im wesentlichen gleich sind.

6. Vorrichtung nach einem der Ansprüche 3 bis 5, **dadurch gekennzeichnet, dass** jede Arbeitselektrode (2,3) einen Ausgang (5) aufweist, der mit dem nicht-invertierenden Eingang (ENI2-j, ENI3-k) des entsprechenden Operationsverstärkers (AO2-j,AO3-k) verbunden ist, der einen Ausgang (S2-j,S3-k) hat, der mit dem entsprechenden Eingang (E2-j,E3-k) der Verarbeitungseinrichtungen (6,8,9) verbunden ist.

7. Vorrichtung nach einem der Ansprüche 3 bis 6, **dadurch gekennzeichnet, dass** die Zusatzelektrode (4) einen Ausgang (5) aufweist, der mit dem nicht-invertierenden Eingang (ENI4) des entsprechenden Operationsverstärkers (AO4) verbunden ist, der einen Ausgang (S4) hat, der mit dem entsprechenden Eingang (E4) der Verarbeitungseinrichtungen (6,8,9) verbunden ist.

8. Vorrichtung nach einem der Ansprüche 3 bis 6, **dadurch gekennzeichnet, dass** die Zusatzelektrode (4) einen Ausgang (5) aufweist, der mit dem Ausgang (S4) des entsprechenden Operationsverstärkers (AO4) verbunden ist, der einen nicht-invertierenden Eingang (ENI4) hat, der mit dem entsprechenden Eingang (E4) der Verarbeitungseinrichtungen (6, 8 9) verbunden und auf dem elektrischen Zwischenpotential gehalten ist, das mit der Versorgungsquelle mittels der Schaltung der Zusatzelektrode (14) verbunden ist.

9. Vorrichtung nach einem der Ansprüche 3 bis 8, **dadurch gekennzeichnet, dass** die Schaltung der Zusatzelektrode (14) einen ersten Widerstand (R1) zwischen einem ersten Endanschluss (11) der Versorgungsquelle (10) und dem nicht-invertierenden Eingang (ENI4) des Operationsverstärkers (AO4) entsprechend der Zusatzelektrode (4) sowie einen zweiten Widerstand (R2) gleichen Wertes wie der erste Widerstand zwischen einem zweiten Endanschluss (12) der genannten Versorgungsquelle (10) und dem nicht-invertierenden Eingang (ENI4) des genannten Operationsverstärkers (AO4) entsprechend der Zusatzelektrode (4) besitzt.

10. Vorrichtung nach einem der Ansprüche 3 bis 8, **dadurch gekennzeichnet, dass** der nicht-invertierende Eingang des Operationsverstärkers direkt mit dem Mittenpunkt der Versorgungsquelle verbunden ist.

11. Vorrichtung nach einem der Ansprüche 3 bis 10, **dadurch gekennzeichnet, dass** der Operationsverstärker (AO4), mit dem die Zusatzelektrode (4) ausgerüstet ist, vom bipolaren Typ ist.

12. Vorrichtung nach einem der Ansprüche 3 bis 10, **dadurch gekennzeichnet, dass** der Operationsverstärker (AO4), mit dem die Zusatzelektrode (4) ausgerüstet ist, vom Feldeffekt-Typ ist.

13. Vorrichtung nach einem der Ansprüche 3 bis 12, **dadurch gekennzeichnet, dass** jeder Operationsverstärker (AO2-j, AO3-k), mit denen die Arbeitselektroden, (2-j,3-k) ausgerüstet sind, vom bipolaren Typ ist.

14. Vorrichtung nach einem der Ansprüche 3 bis 12, **dadurch gekennzeichnet, dass** jeder Operationsverstärker (AO2-j, AO3-k), mit denen die Arbeitselektroden, (2-j,3-k) ausgerüstet sind, vom Feldeffekt-Typ ist.

15. Vorrichtung nach Anspruch 14, **dadurch gekennzeichnet, dass** sie einerseits zwischen dem ersten Endanschluss (11) der Versorgungsquelle (10) und dem nicht-invertierenden Eingang (ENI2-j,ENI3-k) eines jeden Operationsverstärkers (AO2-j,AO3-k) entsprechend jeder Arbeitselektrode (2-j,3-k) eine erste, in Sperrrichtung gepolte Diode (13) und andererseits zwischen dem zweiten Endanschluss (12) der Versorgungsquelle (10) und dem nicht-invertierenden Eingang (ENI2-j,ENI3-k) eines jeden Operationsverstärkers (AO2-j,AO3-k) entsprechend jeder Arbeitselektrode (2-j, 3-k) eine zweite in Sperrrichtung gepoite Diode (13) gleicher Eigenschaften wie die der ersten Diode aufweist.

16. Vorrichtung nach einem der vorhergehenden Ansprüche, **dadurch gekennzeichnet, dass** die Arbeitselektroden (2,3) wenigstens eine Bezugselektrode (3) enthalten.

17. Vorrichtung nach einem der Ansprüche 1 bis 15, **dadurch gekennzeichnet, dass** wenigstens drei Erfassungselektroden vorgesehen sind und dass die Verarbeitungseinrichtungen dazu eingerichtet sind, ein fiktives Bezugspotential aus den von jeder Erfassungselektrode gelieferten Signalen zu berechnen.

18. Vorrichtungen nach einem der vorhergehenden Ansprüche, **dadurch gekennzeichnet, dass** die Versorgungsquelle (10) von einer Zelle oder aus mehreren, in der Reihe geschalteten Zellen oder auch von wiederaufladbaren Akkumulatoren gebildet ist, deren Potentialdifferenz an den Anschlüssen (11,12) im wesentlichen gleich 9 Volt ist.

19. Vorrichtung nach einem der Ansprüche 1 bis 17, **dadurch gekennzeichnet, dass** die Versorgungsquelle (10) von einem Gleichspannungswandler gebildet ist.

20. Vorrichtung nach einem der Ansprüche 1 bis 19, **dadurch gekennzeichnet, dass** die Aufnehmeranordnung (1) ein elektrophysiologischer Helm ist, der an den Schädel des Subjekts (P) anpassbar ist, und dass die von den Erfassungselektroden aufgenommenen Signale Hirnaktivitätswellen sind, die aus identifizierbaren Bereichen des Gehirns stammen.

## Claims

1. Electrophysiological device, of the type comprising sensor equipment (1) which can be fitted to a subject (P) and is equipped with a set of electrodes (2-4) capable of detecting electromagnetic signals and delivering them, in the form of output signals, to inputs (E) of processing means (6, 8, 9) which are capable of shaping them before they are stored, said set comprising working electrodes (2, 3) having at least one acquisition electrode (2) in the chosen position, as well as an additional electrode (4) to be connected to the processing means as a floating earth,
**characterised in that** the output (S2-j, S3-k; j = from 1 to n, k = from 1 to 3) of each working electrode (2-j, 3-k) is equipped with a first impedance adapter means (AO2-j, AO3-k), and
**in that** it comprises a common power supply (10) for those first impedance adapter means (AO2, AO3) as well as a circuit (14) capable of maintaining an intermediate electric potential, with reference to that power supply (10), at the electric potential of the additional electrode (E4).

2. Device according to claim 1, **characterised in that** the output (S4) of the additional electrode (4) is equipped with a second impedance adapter means (AO4) which is also supplied by the power supply (10).

3. Device according to claim 2, **characterised in that** each of the first (AO2, AO3) and second (AO4) impedance adapter means is an operational amplifier comprising inverting inputs (EIi; i = from 2 to 4) and non-inverting inputs (ENIi), as well as an output (Si).

4. Device according to claim 3, **characterised in that** the output (Si) of each operational amplifier (AOi) is coupled to the inverting input (EIi) thereof.

5. Device according to any one of claims 1 to 4, **characterised in that** the respective gains of said first impedance adapter means are substantially equal.

6. Device according to any one of claims 3 to 5, **characterised in that** each working electrode (2, 3) has an output (5) connected to the non-inverting input (ENI2-j, ENI3-k) of the corresponding operational amplifier (AO2-j, AO3-k), which has an output (S2-j, S3-k) connected to the corresponding input (E2-j, E3-k) of the processing means (6, 8, 9).

7. Device according to any one of claims 3 to 6, **characterised in that** the additional electrode (4) has an output (5) connected to the non-inverting input (ENI4) of the corresponding operational amplifier (AO4), which has an output (S4) connected to the corresponding input (E4) of the processing means (6, 8, 9).

8. Device according to any one of claims 3 to 6, **characterised in that** the additional electrode (4) has an output (5) connected to the output (S4) of the corresponding operational amplifier (AO4), which has a non-inverting input (ENI4) which is connected to the corresponding input (E4) of the processing means (6, 8, 9) and is maintained at the intermediate electric potential with reference to the power supply by way of the circuit of the additional electrode (14).

9. Device according to any one of claims 3 to 8, **characterised in that** the circuit of the additional electrode (14) comprises a first resistor (R1) between a first end terminal (11) of the power supply (10) and the non-inverting input (ENI4) of the operational amplifier (AO4) corresponding to the additional electrode (4), as well as a second resistor (R2), of equal value to the first resistor, between a second end terminal (12) of said power supply (10) and the non-inverting input (ENI4) of said operational amplifier (AO4) corresponding to the additional electrode (4).

10. Device according to any one of claims 3 to 8, **characterised in that** the non-inverting input of the operational amplifier is connected directly to the middle point of the power supply.

11. Device according to any one of claims 3 to 10, **characterised in that** the operational amplifier (AO4) with which the additional electrode (4) is equipped is of the bipolar type.

12. Device according to any one of claims 3 to 10, **characterised in that** the operational amplifier (AO4) with which the additional electrode (4) is equipped is of the field-effect type.

13. Device according to any one of claims 3 to 12, **characterised in that** each operational amplifier (AO2-j, AO3-k) with which a working electrode (2-j, 3-k) is equipped is of the bipolar type.

14. Device according to any one of claims 3 to 12, **characterised in that** each operational amplifier (AO2-j, AO3-k) with which a working electrode (2-j, 3-k) is equipped is of the field-effect type.

15. Device according to claim 14, **characterised in that** it comprises, between the first end terminal (11) of the power supply (10) and the non-inverting input (ENI2-j, ENI3-k) of each operational amplifier (AO2-j, AO3-k) corresponding to each working electrode (2-j, 3-k), a first reverse-biased diode (13) and, between the second end terminal (12) of said power supply (10) and the non-inverting input (ENI2-j, ENI3-k) of each operational amplifier (AO2-j, AO3-k) corresponding to each working electrode (2-j, 3-k), a second reverse-biased diode (13) whose characteristics are identical with those of the first diode.

16. Device according to any one of the preceding claims, **characterised in that** the working electrodes (2, 3) comprise at least one reference electrode (3).

17. Device according to any one of claims 1 to 15, **characterised in that** there are at least three acquisition electrodes, and **in that** the processing means are capable of calculating a notional reference potential from the signals delivered by each acquisition electrode.

18. Device according to any one of the preceding claims, **characterised in that** the power supply (10) is constituted by a battery, or by a plurality of batteries connected in series, or alternatively by rechargeable accumulators, the potential difference at the terminals (11, 12) thereof being substantially equal to 9 volts.

19. Device according to any one of claims 1 to 17, **characterised in that** the power supply (10) is constituted by a d.c. converter.

20. Device according to any one of claims 1 to 19, **characterised in that** the sensor equipment (1) is an electrophysiological helmet which can be fitted to the head of the subject (P), and **in that** the signals detected by the acquistion electrodes are waves of cerebral activity coming from identifiable regions of the brain.
